# EUROPEAN PATENT APPLICATION

(11) **EP 4 389 906 A1**
(43) Date of publication of application: **26.06.2024**
(21) Application number: 23153574.1
(22) Date of filing: 27.01.2023
(51) Int. Cl.: C12P 21/00, C12N 9/24, A23K 10/38, C12P 7/06, A23J 1/16

(54) **METHODS FOR THE ENZYMATIC TREATMENT OF WHOLE STILLAGE**

(30) Priority: 21.12.2022 US 202263476433 P
(71) Applicant: BASF SE, 67056 Ludwigshafen am Rhein (DE)
(72) Inventor: Blocquel, David, San Diego, CA 92121 (US); Huffman, James R., San Diego, CA 92121 (US); Qureshi, Asfia, San Diego, CA 92121 (US); Risoer, Michael Wulff, San Diego, CA 92121 (US)
(74) Representative: Maiwald GmbH

(57) **Abstract**

The present invention relates to methods of treating whole stillage with a xylanase to increase the solids content by increasing the protein content in thin stillage.

## Description

### FIELD OF THE INVENTION

The present invention relates to methods of treating whole stillage with a xylanase to increase the solids content in thin stillage.

### BACKGROUND OF THE INVENTION

The first step in the production of fermentation products, such as ethanol, from starch-containing plant material is the degradation of the starch-containing plant material into fermentable sugars by liquefaction and saccharification. These fermentable sugars are then directly or indirectly converted into the desired fermentation product using a fermenting organism such as yeast. Liquid fermentation products such as ethanol are recovered from the fermented mash by methods such as distillation, which separates the desired fermentation product from other liquids and/or solids. The fraction remaining after recovering the liquid fermentation product is referred to as "whole stillage". The whole stillage is dewatered and separated into a solid and a liquid phase, e.g., by centrifugation. The solid phase recovered from whole stillage is referred to as "wet cake" and the liquid phase (supernatant) recovered from whole stillage is referred to as "thin stillage". Dewatered wet cake is dried to provide "Distillers Dried Grains" (DDG) used as nutrient in animal feed. Thin stillage is typically evaporated to provide condensate and syrup or may alternatively be recycled directly to the slurry tank as "backset". The syrup consists mainly of limit dextrins and non-fermentable sugars. It may be blended into DDG or added to the wet cake before drying to produce DDGS (Distillers Dried Grain with Solubles).

It is common to commercially use the various by-products obtained in the fermentation processes such as the ethanol production process, as they are known to provide protein and energy sources for animal feed. Furthermore, the oil from the by-products like DDGS can be recovered as a separate by-product for use in biodiesel production or other biorenewable products. The by-products DDG, DDGS and WDG comprise proteins, fibers, fat and unconverted starch.

As the by-products are used in animal feed, it is important that they have high concentrations of protein. Therefore, it is desirable to increase the protein content of the by-products.

In the prior art, specific processes or treatment methods are described to improve the recovery of by-products.

For example, WO 2007/056321 A1 discloses a method of dewatering whole stillage comprising adding enzymes such as xylanases or cellulases to whole stillage.

Nevertheless, there is still a need for processes which increase the protein content in the by-products of fermentation processes, e.g. in thin stillage.

### SUMMARY OF THE INVENTION

The present invention is directed to a method for increasing the solids content in thin stillage, comprising the steps of:
(a) adding a xylanase to whole stillage, thereby providing a mixture of whole stillage and xylanase; and
(b) incubating the mixture of whole stillage and xylanase; and
(c) separating the whole stillage into thin stillage and wet cake.

In one embodiment, the solids comprise protein.

In one embodiment, the mixture of whole stillage and xylanase is incubated at a temperature of at least 50°C.

In one embodiment, the mixture of whole stillage and xylanase is incubated for one to 24 hours.

In one embodiment, the xylanase is a thermostable xylanase and/or a GH10 xylanase and/or the xylanase has an amino acid sequence which is at least 80% to the amino acid sequence according to SEQ ID NO: 1.

In one embodiment, the xylanase is the only enzyme added to the whole stillage.

In one embodiment, the the whole stillage is derived from a process of producing a fermentation product from a starch-containing material.

In one embodiment, the whole stillage is obtained by the steps of:
(a) saccharifying a starch-containing material;
(b) fermenting the saccharified starch-containing material with a fermenting organism to produce a fermentation product; and
(c) distilling the fermentation product to form the whole stillage.

In one embodiment, the starch-containing material is a cereal, preferably is selected from the group consisting of corn, wheat, barley, cassava, sorghum, rye or potato.

In one embodiment, the fermentation product is an alcohol, preferably is ethanol.

In one embodiment, the whole stillage is separated into thin stillage and wet cake by use of paddle screen.

The method may further comprise a step (d) of isolating protein from thin stillage.

The present invention also relates to the use of a xylanase for increasing the solids content, preferably the protein content, in thin stillage.

### BRIEF DESCRIPTION OF THE DRAWINGS

Figure 1: Protein content in thin stillage after treatment of whole stillage with the GH10 xylanase according to SEQ ID NO: 1
   (a) Total nitrogen content in thin stillage after treatment of whole stillage with the GH10 xylanase according to SEQ ID NO: 1
   (b) Protein increase in thin stillage after treatment of whole stillage with the GH 10 xylanase according to SEQ ID NO: 1
Figure 2: Protein content in thin stillage after treatment of whole stillage with the GH11 xylanase according to SEQ ID NO: 2
   (a) Total nitrogen content in thin stillage after treatment of whole stillage with the GH11 xylanase according to SEQ ID NO: 2
   (b) Protein increase in thin stillage after treatment of whole stillage with the GH 11 xylanase according to SEQ ID NO: 2
Figure 3: Comparison of increase in protein content in thin stillage after treatment of whole stillage with the GH10 xylanase according to SEQ ID NO: 1 and the GH11 xylanase according to SEQ ID NO: 2
Figure 4: Protein content in thin stillage after treatment of whole stillage with the GH 10 xylanase Rohalase
   (a) Total nitrogen content in thin stillage after treatment of whole stillage with the GH10 xylanase according to SEQ ID NO: 3
   (b) Protein increase thin stillage after treatment of whole stillage with the GH 10 xylanase according to SEQ ID NO: 3

### DETAILED DESCRIPTION OF THE INVENTION

The present invention may be understood more readily by reference to the following detailed description of the embodiments of the invention and the examples included herein.

Although the present invention will be described with respect to particular embodiments, this description is not to be construed in a limiting sense.

### Definitions

Unless otherwise noted, the terms used herein are to be understood according to conventional usage by those of ordinary skill in the relevant art.

Before describing in detail exemplary embodiments of the present invention, definitions important for understanding the present invention are given. Unless stated otherwise or apparent from the nature of the definition, the definitions apply to all compounds, methods and uses described herein.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %. Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms "first", "second", "third" or "(a)", "(b)", "(c)", "(d)", "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

Throughout this application, various publications are referenced. The disclosure of all of these publications and those references cited within those publications in their entireties are hereby incorporated by reference into this application in order to more fully describe the state of the art to which this invention pertains.

It is to be understood that the term "comprising" is not limiting. For the purposes of the present invention the term "consisting of" is considered to be a preferred embodiment of the term "comprising". If hereinafter a group is defined to comprise at least a certain number of members, this is meant to also encompass a group which consists of these members only.

The present inventors have surprisingly found that by treating whole stillage with a xylanase, the solids content, in particular the protein content, in thin stillage can be significantly increased. Within the present application, the term "solids" comprises proteins, oil and fibers which are present in thin stillage. One main component of the solids present in thin stillage are proteins so that the method according to the present invention particularly increases the protein content in thin stillage.

The term "increasing the solids content" means that the solids content, in particular the protein content, in thin stillage obtained from whole stillage treated with a xylanase is increased by at least 1%, at least 5%, at least 7%, at least 10%, at least 12%, at least 15%, at least 18%, at least 20%, at least 22%, at least 25%, at least 28%, at least 30% or at least 32% compared to the solids content, in particular the protein content, in thin stillage obtained from whole stillage not treated with a xylanase.

The solids content in thin stillage can be determined by measuring the moisture content as described in the examples herein. Typically, the moisture content is determined by measuring the mass loss upon drying in an oven, an infrared heater or a microwave. The samples are either weighed manually before and after drying or a moisture analyzer is used which is a balance with a heating element which weighs the sample while it dries.

The term "increasing the protein content" means that the protein content in thin stillage obtained from whole stillage treated with a xylanase is increased by at least 1%, at least 5%, at least 7%, at least 10%, at least 12%, at least 15%, at least 18%, at least 20%, at least 22%, at least 25%, at least 28%, at least 30%, at least 32%, at least 34%, at least 36%, at least 38% or at least 40% compared to the protein content in thin stillage obtained from whole stillage not treated with a xylanase.

The protein content in thin stillage can be determined by measuring the amount of nitrogen in thin stillage as described in the examples herein. In one embodiment, the sample is burned and the amount of nitrogen released in the gas is measured, e.g. by a macrocombustion instrument. The term "whole stillage" is known in the art and refers to a by-product obtained after recovery of a fermentation product, preferably by distillation, from fermented mash. Whole stillage consists of liquids and solids which can be separated into thin stillage (i.e. the liquid fraction) and wet cake (i.e. the solid fraction). Whole stillage typically contains about 10-15 wt-% dry solids. Whole stillage components include fiber, hull, germ, oil and protein components from the starch-containing feedstock as well as non-fermented starch.

The term "thin stillage" is known in the art and refers to the liquid phase obtained by separating whole stillage in a liquid and a solid phase. Although it is the liquid phase, the thin stillage may contain a certain amount of solids. As mentioned above, the solids in the thin stillage comprise proteins, fibers and oil, in particular proteins. Thin stillage is typically evaporated to produce condensate (process water) and "Syrup". Condensate can be cleaned in a methanator before being discharged from the plant or it may be added recycled to the front end of the plant and mixed with fresh ground corn in the slurry tank. Syrup, which contains limit dextrins and non-fermentable sugars is added to DDG or wet cake before drying to produce DDGS (Distiller's Dried Grain with Solubles).

The term "wet cake" is known in the art and refers to the solid phase obtained by separating whole stillage in a liquid and a solid phase. The wet cake material is then dried to produce "Distillers Dried Grains" (DDG) which is sold a nutrient in animal feed.

"Xylanases", also called β-1,3- or β-1,4 xylan xylohydrolases, are enzymes which are categorized in Enzyme Class 3.2.1.8 and which depolymerize xylan, arabinoxylan, and/or other xylan-containing polysaccharides. Xylanases may be obtained from any suitable organism, including fungal and bacterial organisms. Fungal xylanases may be derived from strains of genera including Aspergillus such as *Aspergillus tubigensis* (WO 92/01793), *Aspergillus niger* (Shei et al., 1985, Biotech. and Bioeng. Vol. XXVII, pp. 533-538, and Fournier et al., 1985, Biotech. Bioeng. Vol. XXVII, pp. 539-546; WO 91/19782 and EP 463 706) and *Aspergillus aculeatus* (WO 94/21785), Disporofrichum, Penicillium, Neurospora, Fusarium and Trichoderma. Examples of suitable bacterial xylanases include include xylanases derived from a strain of Bacillus, such as *Bacillus subtilis* (US5,306,633) or *Bacillus agaradhaerens* (WO 94/01532), *Bacilluspumilus* (WO 95/182109) or *Bacillus stearothermophilus* (WO 95/182109). Commercially available xylanases include Natugrain TSL, Xylathin HT, Rohalase, Shearzyme^{™}, Biofeed Wheat^{™}, Pulpzyme^{™} HC, BioBrite^{™} EB and Spezyme^{™} CP.

Endo-xylanases (e.g. endo-β-1,4-xylanase) hydrolyze the internal β-glycosidic linkages in xylan, arabinoxylan, and/or other xylose-containing polysaccharides to produce smaller molecular weight xylo-oligomers or xylose monomers. Endo-β-1,4-xylanases are members of the glycoside hydrolase (GH) families 10 and 11. Xylanases of the GH10 and GH11 families differ in their structures, substrate specificity and catalytic mechanism. GH10 xylanases adopt a (β/α)8-barrel fold, while the structures of GH11 xylanases are β-jelly roll. GH10 xylanases have a relatively broad substrate specificity and are able to cleave the β-1,4 glycosidic bond closer to the side chain, whereas GH11 xylanases specifically catalyze xylan degradation. Additionally, GH11 xylanases can easily access complex biomass due to their low molecular weight and high catalytic activity. The GH10 xylanases have a higher molecular mass and a lower pl than the GH11 xylanases (see, e.g., Collins et al. (2005) FEMS Microbiology Reviews 29: 3-23). More information can be found in the CAZYpedia website (Glycoside Hydrolase Family 10 - CAZypedia). In the method of the present invention, preferably a GH10 xylanase is used. GH10 xylanases can be obtained from *Cellulomonas fimi* (Xyn 10A), *Cellvibriojaponicus* (Xyn 10A, Xyn 10C), *Cellvibrio mixtus* (Xyn 10b), *Clostridium thermocellum* NCIB 10682 (xylanase Z), *Geobacillus stearothermophilus* T-6 (xylanase T-6, Xyn A2), *Penicillium simplicissimum* BT2246 (Xyn A), *Streptomyces halstedii*JM8 (Xys 1), *Streptomyces lividans* (xylanase A), *Streptomyces olivaceoviridis* E-86 (beta-1,4 xylanase), *Thermoascus aurantiacus* and *Thermotoga maritima.* Commercially available GH10 xylanases include Natugrain^{®} and Rovabio^{®}. Preferably, the xylanase used in the method of the present invention is a thermostable xylanase. As used herein, "thermostable" means that the xylanase retains a significant amount of its activity (eg, specific activity, relative activity, etc.) within a relevant temperature range for a particular period of time needed for the enzyme to carry out its function. For example, the thermostable xylanase which can be used in the method of the present invention exhibits thermostability at temperatures ranging from about 75°C to 100°C, preferably from about 80°C to 95°C, more preferably from about 82°C to 92°C, or about 85°C, about 88°C or about 90°C. The term "thermostable" encompasses enzymes that have a temperature optimum within the relevant temperature range as well as enzymes with higher or lower temperature optima, as long as the enzyme retains a significant amount of its activity within the relevant temperature range over the relevant time period (e.g., they retain their activity for a period of 2 hours at a temperature of 84°C). The thermostable xylanase retains at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% of their activity when used for 1 to 24 hours, preferably 2 to 12 hours or 2 to 8 hours at a temperature of at least 75°C, at least 80°C, at least 82°C or 84°C. The thermostable xylanase preferably has a Melting Point (Tm) from about 75°C to about 110°C, for example above about 80°C, about 82°C, about 84°C, about 85°C , about 86°C, about 88°C, about 90°C, about 92°C, about 94°C, about 95°C, about 96°C, about 98°C, about 100°C, about 105°C, or up to about 110°C. The melting point may be determined by techniques available to the skilled artisan, for example by a Differential Scanning Calorimetry assay (DSC). In addition, activity assays can be used to determine the activity of the xylanase at different temperatures to determine the temperature optimum of the thermostable xylanase, as well as to determine how much activity the thermostable xylanase retains within a particular temperature range or over a period of time.

In the method of the present invention, preferably a thermostable GH10 xylanase is used.

In a particular embodiment, the xylanase used in the method of the present invention has an amino acid sequence which is at least 80%, at least 81%, at least 82%, at least 83%, at least 84%, at least 85%, at least 86%, at least 87%, at least 88%, at least 89%, at least 90%, at least 90.5%, at least 91%, at least 91.5%, at least 92%, at least 92.5%, at least 93%, at least 93.5%, at least 94%, at least 94.5%, at least 95%, at least 95.1%, at least 95.2%, at least 95.3%, at least 95.4%, at least 95.5%, at least 95.6%, at least 95.7%, at least 95.8%, at least 95.9%, at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% identical to the amino acid sequence according to SEQ ID NO: 1. Sequence identity usually is provided as "% sequence identity" or "% identity". For calculation of sequence identities, in a first step a sequence alignment is produced. According to this invention, a pairwise global alignment is produced, meaning that two sequences are aligned over their complete length, which is usually produced by using a mathematical approach, called alignment algorithm.

According to the invention, the alignment is generated by using the algorithm of Needleman and Wunsch (J. Mol. Biol. (1979) 48, p. 443-453). Preferably, the program "NEEDLE" (The European Molecular Biology Open Software Suite (EMBOSS)) is used for the purposes of the current invention, with using the programs default parameter (polynucleotides: gap open=10.0, gap extend=0.5 and matrix=EDNAFULL; polypeptides: gap open=10.0, gap extend=0.5 and matrix=EBLOSUM62). After aligning two sequences, in a second step, an identity value is determined from the alignment produced. For this purpose, the %-identity is calculated by dividing the number of identical residues by the length of the alignment region which is showing the respective sequence of the present invention over its complete length multiplied with 100: %-identity = (identical residues / length of the alignment region which is showing the respective sequence of the present invention over its complete length) *100.

The xylanase may be added to the whole stillage as an isolated enzyme or as part of a mixture containing additional enzymes. In a preferred embodiment, the xylanase is the only enzyme added to the whole stillage, i.e. it is added as an isolated enzyme and not as part of a mixture with other enzymes. In one embodiment, the xylanase is added to whole stillage after the completion of fermentation and distilling the fermentation product.

The xylanase may be added to the whole stillage at a concentration of 10 to 1000 ppm, preferably of 10 to 400 ppm.

Once the mixture of xylanase and whole stillage is formed, it is incubated under suitable conditions, i.e. under conditions which allow the xylanase to exert its enzymatic activity on the whole stillage.

In one embodiment, the mixture of xylanase and whole stillage is incubated at a temperature of at least 50°C, at least 55°C, at least 60°C, at least 65°C, at least 70°C, at least 72°C, at least 74°C, at least 76°C, at least 78°C, at least 80°C, at least 81°C, at least 82°C, at least 83°C, at least 84°C or at least 85°C. In one embodiment, the mixture of xylanase and whole stillage is incubated at a temperature of 50°C to 100°C, 55°C to 100°C, 60°C to 100°C, 65°C to 100°C, 70°C to 100°C, 72°C to 100°C, 74°C to 100°C, 76°C to 100°C, 78°C to 100°C, 80°C to 100°C, 81°C to 100°C, 82°C to 100°C, 83°C to 100°C, 84°C to 100°C or 85°C to 100°C. In one embodiment, the mixture of xylanase and whole stillage is incubated at a temperature of 50°C to 95°C, 50°C to 93°C, 50°C to 90°C, 50°C to 88°C, 50°C to 86°C, 50°C to 85°C, 50°C to 84°C, 50°C to 83°C, 50°C to 82°C, 50°C to 81°C or 50°C to 80°C. In one embodiment, the mixture of xylanase and whole stillage is incubated at a temperature of 70°C to 90°C, 71°C to 89°C, 72°C to 88°C, 73°C to 87°C, 74°C to 86°C, 73°C to 85°C, 74°C to 85°C, 75°C to 85°C, 76°C to 85°C, 77°C to 85°C or 78°C to 85°C. In one embodiment, the mixture of xylanase and whole stillage is incubated at a temperature of 80°C to 90°C. In one embodiment, the mixture of xylanase and whole stillage is incubated at a temperature of 80°C, 81°C, 82°C, 83°C, 84°C, 85°C, 86°C, 87°C, 88°C, 89°C or 90°C. In a particular embodiment, the mixture of xylanase and whole stillage is incubated at a temperature of 84°C.

In one embodiment, the mixture of xylanase and whole stillage is incubated for a period of one to 24 hours, one to 20 hours, one to 18 hours, one to 16 hours, one to 14 hours, one to twelve hours, one to ten hours, one to nine hours, one to eight hours, one to seven hours, one to six hours, one to five hours, one to four hours or one to three hours. In one embodiment, the mixture of xylanase and whole stillage is incubated for a period of one hour, two hours, three hours, four hours, five hours, six hours, seven hours, eight hours, nine hours, ten hours, eleven hours, twelve hours, 13 hours, 14 hours, 15 hours, 16 hours, 17 hours, 18 hours, 19 hours, 20 hours, 21 hours, 22 hours, 23 hours or 24 hours. In a particular embodiment, the mixture of xylanase and whole stillage is incubated for a period of two hours.

In one embodiment, the mixture of xylanase and whole stillage is incubated at a pH of 4.0 to 6.0, 4.1 to 5.9, 4.2 to 5.8, 4.3 to 5.7, 4.4 to 5.6, 4.5 to 5.5, 4.6 to 5.4, 4.7 to 5.3, 4.8 to 5.2 or 4.9 to 5.1. In one embodiment, the mixture of xylanase and whole stillage is incubated at a pH of 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9 or 6.0. In a particular embodiment, the mixture of xylanase and whole stillage is incubated at a pH of 5.0.

In one embodiment, the mixture of xylanase and whole stillage is incubated at a temperature of 50°C to 100°C and a pH of 4.0 to 6.0. In one embodiment, the mixture of xylanase and whole stillage is incubated at a temperature of 70°C to 100°C and a pH of 4.2 to 5.8. In one embodiment, the mixture of xylanase and whole stillage is incubated at a temperature of 70°C to 90°C and a pH of 4.5 to 5.5. In one embodiment, the mixture of xylanase and whole stillage is incubated at a temperature of 80°C to 90°C and a pH of 4.8 to 5.2. In one embodiment, the mixture of xylanase and whole stillage is incubated at a temperature of 84°C and a pH of 5.0.

In one embodiment, the mixture of xylanase and whole stillage is incubated at a temperature of 50°C to 100°C for a period of 1 to 24 hours. In one embodiment, the mixture of xylanase and whole stillage is incubated at a temperature of 70°C to 100°C for a period of 1 to 12 hours. In one embodiment, the mixture of xylanase and whole stillage is incubated at a temperature of 70°C to 90°C for a period of 1 to eight hours. In one embodiment, the mixture of xylanase and whole stillage is incubated at a temperature of 80°C to 90°C for a period of 1 to six hours. In one embodiment, the mixture of xylanase and whole stillage is incubated at a temperature of 84°C for a period of two hours.

In one embodiment, the mixture of xylanase and whole stillage is incubated at a pH of 4.0 to 6.0 for a period of 1 to 24 hours. In one embodiment, the mixture of xylanase and whole stillage is incubated at a pH of 4.2 to 5.8 for a period of 1 to 12 hours. In one embodiment, the mixture of xylanase and whole stillage is incubated at a pH of 4.5 to 5.5 for a period of 1 to eight hours. In one embodiment, the mixture of xylanase and whole stillage is incubated at a pH of 4.8 to 5.2 for a period of 1 to six hours. In one embodiment, the mixture of xylanase and whole stillage is incubated at a pH of 5.0 for a period of two hours.

In one embodiment, the mixture of xylanase and whole stillage is incubated at a temperature of 50°C to 100°C and a pH of 4.0 to 6.0 for a period of 1 to 24 hours. In one embodiment, the mixture of xylanase and whole stillage is incubated at a temperature of 70°C to 100°C and a pH of 4.2 to 5.8 for a period of 1 to 12 hours. In one embodiment, the mixture of xylanase and whole stillage is incubated at a temperature of 70°C to 90°C and a pH of 4.5 to 5.5 for a period of 1 to eight hours. In one embodiment, the mixture of xylanase and whole stillage is incubated at a temperature of 80°C to 90°C and a pH of 4.8 to 5.2 for a period of 1 to six hours. In one embodiment, the mixture of xylanase and whole stillage is incubated at a temperature of 84°C and a pH of 5.0 for a period of two hours.

Thus, in one embodiment the present invention relates to a method for increasing the solids content in thin stillage, comprising the steps of:
(a) adding a xylanase to whole stillage, thereby providing a mixture of whole stillage and xylanase; and
(b) incubating the mixture of whole stillage and xylanase at a temperature of 50°C to 100°C and a pH of 4.0 to 6.0; and
(c) separating the whole stillage into thin stillage and wet cake,
wherein the xylanase is a thermostable GH10 xylanase.

Thus, in one embodiment the present invention relates to a method for increasing the solids content in thin stillage, comprising the steps of:
(a) adding a xylanase to whole stillage, thereby providing a mixture of whole stillage and xylanase; and
(b) incubating the mixture of whole stillage and xylanase at a temperature of 50°C to 100°C for a period of 1 to 24 hours; and
(c) separating the whole stillage into thin stillage and wet cake,
wherein the xylanase is a thermostable GH10 xylanase.

Thus, in one embodiment the present invention relates to a method for increasing the solids content in thin stillage, comprising the steps of:
(a) adding a xylanase to whole stillage, thereby providing a mixture of whole stillage and xylanase; and
(b) incubating the mixture of whole stillage and xylanase at a temperature of 50°C to 100°C and a pH of 4.0 to 6.0 for a period of 1 to 24 hours; and
(c) separating the whole stillage into thin stillage and wet cake,
wherein the xylanase is a thermostable GH10 xylanase.

Thus, in one embodiment the present invention relates to a method for increasing the solids content in thin stillage, comprising the steps of:
(a) adding a xylanase to whole stillage, thereby providing a mixture of whole stillage and xylanase; and
(b) incubating the mixture of whole stillage and xylanase at a temperature of 80°C to 90°C and a pH of 4.8 to 5.2.; and
(c) separating the whole stillage into thin stillage and wet cake,
wherein the xylanase is a thermostable GH10 xylanase.

Thus, in one embodiment the present invention relates to a method for increasing the solids content in thin stillage, comprising the steps of:
(a) adding a xylanase to whole stillage, thereby providing a mixture of whole stillage and xylanase; and
(b) incubating the mixture of whole stillage and xylanase at a temperature of 80°C to 90°C for a period of 1 to six hours; and
(c) separating the whole stillage into thin stillage and wet cake,
wherein the xylanase is a thermostable GH10 xylanase.

Thus, in one embodiment the present invention relates to a method for increasing the solids content in thin stillage, comprising the steps of:
(a) adding a xylanase to whole stillage, thereby providing a mixture of whole stillage and xylanase; and
(b) incubating the mixture of whole stillage and xylanase at a temperature of 80°C to 90°C and a pH of 4.8 to 5.2 for a period of 1 to six hours; and
(c) separating the whole stillage into thin stillage and wet cake,
wherein the xylanase is a thermostable GH10 xylanase.

Thus, in one embodiment the present invention relates to a method for increasing the solids content in thin stillage, comprising the steps of:
(a) adding a xylanase to whole stillage, thereby providing a mixture of whole stillage and xylanase; and
(b) incubating the mixture of whole stillage and xylanase at a temperature of 50°C to 100°C and a pH of 4.0 to 6.0; and
(c) separating the whole stillage into thin stillage and wet cake,
wherein the xylanase has an amino acid sequence which is at least 80% identical to the amino acid sequence according to SEQ ID NO: 1.

Thus, in one embodiment the present invention relates to a method for increasing the solids content in thin stillage, comprising the steps of:
(a) adding a xylanase to whole stillage, thereby providing a mixture of whole stillage and xylanase; and
(b) incubating the mixture of whole stillage and xylanase at a temperature of 50°C to 100°C for a period of 1 to 24 hours; and
(c) separating the whole stillage into thin stillage and wet cake,
wherein the xylanase has an amino acid sequence which is at least 80% identical to the amino acid sequence according to SEQ ID NO: 1.

Thus, in one embodiment the present invention relates to a method for increasing the solids content in thin stillage, comprising the steps of:
(a) adding a xylanase to whole stillage, thereby providing a mixture of whole stillage and xylanase; and
(b) incubating the mixture of whole stillage and xylanase at a temperature of 50°C to 100°C and a pH of 4.0 to 6.0 for a period of 1 to 24 hours; and
(c) separating the whole stillage into thin stillage and wet cake,
wherein the xylanase has an amino acid sequence which is at least 80% identical to the amino acid sequence according to SEQ ID NO: 1.

Thus, in one embodiment the present invention relates to a method for increasing the solids content in thin stillage, comprising the steps of:
(a) adding a xylanase to whole stillage, thereby providing a mixture of whole stillage and xylanase; and
(b) incubating the mixture of whole stillage and xylanase at a temperature of 80°C to 90°C and a pH of 4.8 to 5.2.; and
(c) separating the whole stillage into thin stillage and wet cake,
wherein the xylanase has an amino acid sequence which is at least 80% identical to the amino acid sequence according to SEQ ID NO: 1.

Thus, in one embodiment the present invention relates to a method for increasing the solids content in thin stillage, comprising the steps of:
(a) adding a xylanase to whole stillage, thereby providing a mixture of whole stillage and xylanase; and
(b) incubating the mixture of whole stillage and xylanase at a temperature of 80°C to 90°C for a period of 1 to six hours; and
(c) separating the whole stillage into thin stillage and wet cake,
wherein the xylanase has an amino acid sequence which is at least 80% identical to the amino acid sequence according to SEQ ID NO: 1.

Thus, in one embodiment the present invention relates to a method for increasing the solids content in thin stillage, comprising the steps of:
(a) adding a xylanase to whole stillage, thereby providing a mixture of whole stillage and xylanase; and
(b) incubating the mixture of whole stillage and xylanase at a temperature of 80°C to 90°C and a pH of 4.8 to 5.2 for a period of 1 to six hours; and
(c) separating the whole stillage into thin stillage and wet cake,
wherein the xylanase has an amino acid sequence which is at least 80% identical to the amino acid sequence according to SEQ ID NO: 1.

Thus, in one embodiment the present invention relates to a method for increasing the solids content in thin stillage, comprising the steps of:
(a) adding a xylanase to whole stillage, thereby providing a mixture of whole stillage and xylanase; and
(b) incubating the mixture of whole stillage and xylanase at a temperature of 84°C and a pH of 5.0; and
(c) separating the whole stillage into thin stillage and wet cake,
wherein the xylanase has the amino acid sequence according to SEQ ID NO: 1.

Thus, in one embodiment the present invention relates to a method for increasing the solids content in thin stillage, comprising the steps of:
(a) adding a xylanase to whole stillage, thereby providing a mixture of whole stillage and xylanase; and
(b) incubating the mixture of whole stillage and xylanase at a temperature of 84°C for a period of 2 hours; and
(c) separating the whole stillage into thin stillage and wet cake,
wherein the xylanase has the amino acid sequence according to SEQ ID NO: 1.

Thus, in one embodiment the present invention relates to a method for increasing the solids content in thin stillage, comprising the steps of:
(a) adding a xylanase to whole stillage, thereby providing a mixture of whole stillage and xylanase; and
(b) incubating the mixture of whole stillage and xylanase at a temperature of 84°C and a pH of 5.0 for a period of 2 hours; and
(c) separating the whole stillage into thin stillage and wet cake,
wherein the xylanase has an the amino acid sequence according to SEQ ID NO: 1.

Thus, in one embodiment the present invention relates to a method for increasing the protein content in thin stillage, comprising the steps of:
(a) adding a xylanase to whole stillage, thereby providing a mixture of whole stillage and xylanase; and
(b) incubating the mixture of whole stillage and xylanase at a temperature of 50°C to 100°C and a pH of 4.0 to 6.0; and
(c) separating the whole stillage into thin stillage and wet cake,
wherein the xylanase is a thermostable GH10 xylanase.

Thus, in one embodiment the present invention relates to a method for increasing the protein content in thin stillage, comprising the steps of:
(a) adding a xylanase to whole stillage, thereby providing a mixture of whole stillage and xylanase; and
(b) incubating the mixture of whole stillage and xylanase at a temperature of 50°C to 100°C for a period of 1 to 24 hours; and
(c) separating the whole stillage into thin stillage and wet cake,
wherein the xylanase is a thermostable GH10 xylanase.

Thus, in one embodiment the present invention relates to a method for increasing the protein content in thin stillage, comprising the steps of:
(a) adding a xylanase to whole stillage, thereby providing a mixture of whole stillage and xylanase; and
(b) incubating the mixture of whole stillage and xylanase at a temperature of 50°C to 100°C and a pH of 4.0 to 6.0 for a period of 1 to 24 hours; and
(c) separating the whole stillage into thin stillage and wet cake,
wherein the xylanase is a thermostable GH10 xylanase.

Thus, in one embodiment the present invention relates to a method for increasing the protein content in thin stillage, comprising the steps of:
(a) adding a xylanase to whole stillage, thereby providing a mixture of whole stillage and xylanase; and
(b) incubating the mixture of whole stillage and xylanase at a temperature of 80°C to 90°C and a pH of 4.8 to 5.2.; and
(c) separating the whole stillage into thin stillage and wet cake,
wherein the xylanase is a thermostable GH10 xylanase.

Thus, in one embodiment the present invention relates to a method for increasing the protein content in thin stillage, comprising the steps of:
(a) adding a xylanase to whole stillage, thereby providing a mixture of whole stillage and xylanase; and
(b) incubating the mixture of whole stillage and xylanase at a temperature of 80°C to 90°C for a period of 1 to six hours; and
(c) separating the whole stillage into thin stillage and wet cake,
wherein the xylanase is a thermostable GH10 xylanase.

Thus, in one embodiment the present invention relates to a method for increasing the protein content in thin stillage, comprising the steps of:
(a) adding a xylanase to whole stillage, thereby providing a mixture of whole stillage and xylanase; and
(b) incubating the mixture of whole stillage and xylanase at a temperature of 80°C to 90°C and a pH of 4.8 to 5.2 for a period of 1 to six hours; and
(c) separating the whole stillage into thin stillage and wet cake,
wherein the xylanase is a thermostable GH10 xylanase.

Thus, in one embodiment the present invention relates to a method for increasing the protein content in thin stillage, comprising the steps of:
(a) adding a xylanase to whole stillage, thereby providing a mixture of whole stillage and xylanase; and
(b) incubating the mixture of whole stillage and xylanase at a temperature of 50°C to 100°C and a pH of 4.0 to 6.0; and
(c) separating the whole stillage into thin stillage and wet cake,
wherein the xylanase has an amino acid sequence which is at least 80% identical to the amino acid sequence according to SEQ ID NO: 1.

Thus, in one embodiment the present invention relates to a method for increasing the protein content in thin stillage, comprising the steps of:
(a) adding a xylanase to whole stillage, thereby providing a mixture of whole stillage and xylanase; and
(b) incubating the mixture of whole stillage and xylanase at a temperature of 50°C to 100°C for a period of 1 to 24 hours; and
(c) separating the whole stillage into thin stillage and wet cake,
wherein the xylanase has an amino acid sequence which is at least 80% identical to the amino acid sequence according to SEQ ID NO: 1.

Thus, in one embodiment the present invention relates to a method for increasing the protein content in thin stillage, comprising the steps of:
(a) adding a xylanase to whole stillage, thereby providing a mixture of whole stillage and xylanase; and
(b) incubating the mixture of whole stillage and xylanase at a temperature of 50°C to 100°C and a pH of 4.0 to 6.0 for a period of 1 to 24 hours; and
(c) separating the whole stillage into thin stillage and wet cake,
wherein the xylanase has an amino acid sequence which is at least 80% identical to the amino acid sequence according to SEQ ID NO: 1.

Thus, in one embodiment the present invention relates to a method for increasing the protein content in thin stillage, comprising the steps of:
(a) adding a xylanase to whole stillage, thereby providing a mixture of whole stillage and xylanase; and
(b) incubating the mixture of whole stillage and xylanase at a temperature of 80°C to 90°C and a pH of 4.8 to 5.2.; and
(c) separating the whole stillage into thin stillage and wet cake,
wherein the xylanase has an amino acid sequence which is at least 80% identical to the amino acid sequence according to SEQ ID NO: 1.

Thus, in one embodiment the present invention relates to a method for increasing the protein content in thin stillage, comprising the steps of:
(a) adding a xylanase to whole stillage, thereby providing a mixture of whole stillage and xylanase; and
(b) incubating the mixture of whole stillage and xylanase at a temperature of 80°C to 90°C for a period of 1 to six hours; and
(c) separating the whole stillage into thin stillage and wet cake,
wherein the xylanase has an amino acid sequence which is at least 80% identical to the amino acid sequence according to SEQ ID NO: 1.

Thus, in one embodiment the present invention relates to a method for increasing the protein content in thin stillage, comprising the steps of:
(a) adding a xylanase to whole stillage, thereby providing a mixture of whole stillage and xylanase; and
(b) incubating the mixture of whole stillage and xylanase at a temperature of 80°C to 90°C and a pH of 4.8 to 5.2 for a period of 1 to six hours; and
(c) separating the whole stillage into thin stillage and wet cake,
wherein the xylanase has an amino acid sequence which is at least 80% identical to the amino acid sequence according to SEQ ID NO: 1.

Thus, in one embodiment the present invention relates to a method for increasing the protein content in thin stillage, comprising the steps of:
(a) adding a xylanase to whole stillage, thereby providing a mixture of whole stillage and xylanase; and
(b) incubating the mixture of whole stillage and xylanase at a temperature of 84°C and a pH of 5.0; and
(c) separating the whole stillage into thin stillage and wet cake,
wherein the xylanase has the amino acid sequence according to SEQ ID NO: 1.

Thus, in one embodiment the present invention relates to a method for increasing the protein content in thin stillage, comprising the steps of:
(a) adding a xylanase to whole stillage, thereby providing a mixture of whole stillage and xylanase; and
(b) incubating the mixture of whole stillage and xylanase at a temperature of 84°C for a period of 2 hours; and
(c) separating the whole stillage into thin stillage and wet cake,
wherein the xylanase has the amino acid sequence according to SEQ ID NO: 1.

Thus, in one embodiment the present invention relates to a method for increasing the protein content in thin stillage, comprising the steps of:
(a) adding a xylanase to whole stillage, thereby providing a mixture of whole stillage and xylanase; and
(b) incubating the mixture of whole stillage and xylanase at a temperature of 84°C and a pH of 5.0 for a period of 2 hours; and
(c) separating the whole stillage into thin stillage and wet cake,
wherein the xylanase has an the amino acid sequence according to SEQ ID NO: 1.

After incubating the mixture of whole stillage and xylanase, the whole stillage is separated into thin stillage and wet cake. The separation of whole stillage into thin stillage and wet cake can be performed by any method known in the art, including decanting, centrifugation such as centrifugation in a decanter centrifuge, and filtration such as filtration using a filter press, a screw press, a plate-and-frame press, a gravity thickener, a paddle screen or decker. Suitable methods for separating whole stillage into thin stillage and wet cake are disclosed in US 6,433,146, US 7,601,858, US 7,608,729, WO 2016/137641 and US 2010/0058649. In the method of the present invention, the whole stillage is preferably separated into thin stillage and wet cake by a paddle screen.

Paddle screen devices include rotating paddles with a stationary drum including an outer wall configured as a screen. The rotation of the paddles directs the slurry toward the screened outer wall and essentially presses the slurry so as to force the water, the starch, and the gluten through the screen while preventing the fiber from passing therethrough. The movement of the paddles relative to the drum loosens the fiber from the outer wall and reduces plugging of the screen openings. The centrifugal force created by the rotating paddles provides a high filtration pressure and consequently a higher capacity per unit screen surface. A suitable paddle screen apparatus is described in WO 2016/137641 A1.

In one embodiment, the method of the present invention further comprises a step (d) of isolating protein from thin stillage. Protein can be isolated from thin stillage by various techniques including, but not limited to, dissolved air flotation (DAF) which is used to flocculate and float the protein from the surface of the liquid, high speed centrifugation which is used to spin the solid material in a tricanter or other high speed centrifugal device or filtration. In a preferred embodiment, the protein is isolated from thin stillage by dissolved air flotation. A suitable isolation method by dissolved air flotation is disclosed in US 7,497,955 B2, US 9,776,105 B2, US 2020/255307 and US 2020/154730.

The method of the present invention may be used on whole stillage derived from production of any suitable fermentation product. Suitable fermentation products include, but are not limited to, alcohols such as ethanol, methanol, butanol, 1,3-propanediol; organic acids such as citric acid, acetic acid, itaconic acid, lactic acid, gluconic acid, gluconate, succinic acid, 2,5-diketo-D-gluconic acid; ketones such as acetone); amino acids such as glutamic acid; gases such as H₂ and CO₂, and more complex compounds, including, for example, antibiotics such as penicillin and tetracycline; enzymes; vitamins such as riboflavin, vitamin B12 and beta-carotene; and hormones. In a particular embodiment, the fermentation product is an alcohol, preferably ethanol.

The fermentation product may be produced from a starch-containing material, preferably from a starch-containing plant material. Starch-containing plant material includes tubers, roots and whole grain and any combination thereof. Suitable plants for obtaining the starch-containing material are cereals such as corn (maize), wheat, barley, cassava, sorghum or rye or potato, or any combination thereof. Preferably, corn is used for obtaining the starch-containing material, in particular when the fermentation product is ethanol.

The whole stillage which is incubated with the xylanase may be obtained by the steps of:
(a) liquefying a starch-containing material;
(b) fermenting the saccharified starch-containing material with a fermenting organism to produce a fermentation product; and
(c) distilling the fermentation product to form the whole stillage.

"Liquefaction" or "liquefying" is a process for gelatinizing and debranching starch with suitable enzymes such as alpha-amylase, and glucoamylase and optionally a debranching enzyme, such as an isoamylase or a pullulanase. Liquefaction is typically carried out at a temperature from 80°C to 95°C, preferably from 85-90°C, typically around 87°C, and at a pH between 4 and 5.5, normally at about pH 5. A full liquefaction process may last from about 1.5 to about 2.5 hours.

Typically, after liquefaction a complete saccharification during fermentation is performed in a simultaneous saccharification and fermentation process (SSF process).

Hence, in one embodiment the whole stillage which is incubated with the xylanase may be obtained by the steps of:
(a) liquefying a starch-containing material;
(b) subjecting the liquefied starch-containing material to a simultaneous saccharification and fermentation process with a fermenting organism to produce a fermentation product; and
(c) distilling the fermentation product to form the whole stillage.

The most widely used process to produce a fermentation product, especially ethanol, is the simultaneous saccharification and fermentation (SSF) process, in which there is no holding stage for the saccharification, meaning that a fermenting organism, such as a yeast, and enzyme(s) used for saccharification, such as glucoamylase, may be added together. In one embodiment, the simultaneous saccharification and fermentation process does not comprise the addition of a xylanase. SSF is typically carried out at a temperature from 25°C to 40°C, such as from 28°C to 35°C, from 30°C to 34°C, preferably around about 32°C.

In one embodiment, fermentation or simultaneous saccharification and fermentation (SSF) is ongoing for 6 to 120 hours, in particular 24 to 96 hours or 48 to 72 hours. In the fermentation step the sugars are converted into the desired fermentation product such as ethanol by the fermenting organism.

In one embodiment, the saccharifying and fermenting or the simultaneous saccharification and fermentation (SSF) is performed in the absence of a xylanase. In this case, the xylanase is only added to the whole stillage and not present in the preceding steps of obtaining the whole stillage.

In the distilling step, the mixture obtained by fermentation or SSF is heated to a temperature of about 90°C to evaporate ethanol and other liquid products. In this step, the whole stillage is separated from the ethanol and/or other fermentation products.

In one embodiment, the whole stillage which is incubated with the xylanase may be obtained by the steps of:
(a) liquefying corn;
(b) subjecting the liquefied corn to a simultaneous saccharification and fermentation process with yeast to produce ethanol; and
(c) distilling the ethanol to form the whole stillage.

Before liquefaction, the starch-containing material such as corn is soaked with water to create a slurry and then treated with alpha-amylase and optionally further enzymes in the liquefaction step. The liquefaction is typically performed at a temperature of 85°C to 90°C for a period of about two hours.

Hence, in one embodiment the method for increasing the solids content in thin stillage, comprises the steps of:
(a) liquefying a starch-containing material;
(b) subjecting the liquefied starch-containing material to a simultaneous saccharification and fermentation process with a fermenting organism to produce a fermentation product;
(c) distilling the fermentation product to form the whole stillage;
(d) adding a xylanase to whole stillage, thereby providing a mixture of whole stillage and xylanase; and
(e) incubating the mixture of whole stillage and xylanase; and
(f) separating the whole stillage into thin stillage and wet cake.

In one embodiment the method for increasing the solids content in thin stillage, comprises the steps of:
(a) liquefying a starch-containing material;
(b) subjecting the liquefied starch-containing material to a simultaneous saccharification and fermentation process with a fermenting organism to produce a fermentation product;
(c) distilling the fermentation product to form the whole stillage;
(d) adding a xylanase to whole stillage, thereby providing a mixture of whole stillage and xylanase; and
(e) incubating the mixture of whole stillage and xylanase; and
(f) separating the whole stillage into thin stillage and wet cake,
wherein the xylanase is a thermostable xylanase.

In one embodiment the method for increasing the solids content in thin stillage, comprises the steps of:
(a) liquefying a starch-containing material;
(b) subjecting the liquefied starch-containing material to a simultaneous saccharification and fermentation process with a fermenting organism to produce a fermentation product;
(c) distilling the fermentation product to form the whole stillage;
(d) adding a xylanase to whole stillage, thereby providing a mixture of whole stillage and xylanase; and
(e) incubating the mixture of whole stillage and xylanase; and
(f) separating the whole stillage into thin stillage and wet cake,
wherein the xylanase is a thermostable GH10 xylanase.

In one embodiment the method for increasing the solids content in thin stillage, comprises the steps of:
(a) liquefying a starch-containing material;
(b) subjecting the liquefied starch-containing material to a simultaneous saccharification and fermentation process with a fermenting organism to produce a fermentation product;
(c) distilling the fermentation product to form the whole stillage;
(d) adding a xylanase to whole stillage, thereby providing a mixture of whole stillage and xylanase; and
(e) incubating the mixture of whole stillage and xylanase; and
(f) separating the whole stillage into thin stillage and wet cake,
wherein the xylanase has an amino acid sequence which is at least 80% identical to the amino acid sequence according to SEQ ID NO: 1.

In one embodiment the method for increasing the solids content in thin stillage, comprises the steps of:
(a) liquefying a starch-containing material;
(b) subjecting the liquefied starch-containing material to a simultaneous saccharification and fermentation process with a fermenting organism to produce a fermentation product;
(c) distilling the fermentation product to form the whole stillage;
(d) adding a xylanase to whole stillage, thereby providing a mixture of whole stillage and xylanase; and
(e) incubating the mixture of whole stillage and xylanase; and
(f) separating the whole stillage into thin stillage and wet cake,
wherein the xylanase has the amino acid sequence according to SEQ ID NO: 1.

In one embodiment, the method for increasing the solids content in thin stillage, comprises the steps of:
(a) liquefying corn;
(b) subjecting the liquefied corn to a simultaneous saccharification and fermentation process with yeast to produce ethanol;
(c) distilling the ethanol to form the whole stillage;
(d) adding a xylanase to whole stillage, thereby providing a mixture of whole stillage and xylanase; and
(e) incubating the mixture of whole stillage and xylanase at a temperature of 84°C and a pH of 5.0 for a period of 2 hours; and
(f) separating the whole stillage into thin stillage and wet cake,
wherein the xylanase is a thermostable xylanase.

In one embodiment, the method for increasing the solids content in thin stillage, comprises the steps of:
(a) liquefying corn;
(b) subjecting the liquefied corn to a simultaneous saccharification and fermentation process with yeast to produce ethanol;
(c) distilling the ethanol to form the whole stillage;
(d) adding a xylanase to whole stillage, thereby providing a mixture of whole stillage and xylanase; and
(e) incubating the mixture of whole stillage and xylanase at a temperature of 84°C and a pH of 5.0 for a period of 2 hours; and
(f) separating the whole stillage into thin stillage and wet cake,
wherein the xylanase is a thermostable GH10 xylanase.

In one embodiment, the method for increasing the solids content in thin stillage, comprises the steps of:
(a) liquefying corn;
(b) subjecting the liquefied corn to a simultaneous saccharification and fermentation process with yeast to produce ethanol;
(c) distilling the ethanol to form the whole stillage;
(d) adding a xylanase to whole stillage, thereby providing a mixture of whole stillage and xylanase; and
(e) incubating the mixture of whole stillage and xylanase at a temperature of 84°C and a pH of 5.0 for a period of 2 hours; and
(f) separating the whole stillage into thin stillage and wet cake,
wherein the xylanase has an amino acid sequence which is at least 80% identical to the amino acid sequence according to SEQ ID NO: 1.

In one embodiment, the method for increasing the solids content in thin stillage, comprises the steps of:
(a) liquefying corn;
(b) subjecting the liquefied corn to a simultaneous saccharification and fermentation process with yeast to produce ethanol;
(c) distilling the ethanol to form the whole stillage;
(d) adding a xylanase to whole stillage, thereby providing a mixture of whole stillage and xylanase; and
(e) incubating the mixture of whole stillage and xylanase at a temperature of 84°C and a pH of 5.0 for a period of 2 hours; and
(f) separating the whole stillage into thin stillage and wet cake,
wherein the xylanase has the amino acid sequence according to SEQ ID NO: 1.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims. The detailed description is merely exemplary in nature and is not intended to limit application and uses. The following examples further illustrate the present invention without, however, limiting the scope of the invention thereto. Various changes and modifications can be made by those skilled in the art on the basis of the description of the invention, and such changes and modifications are also included in the present invention.

### EXAMPLES

### Materials and methods

Aliquots of 20mL of Corn Whole stillage were treated with different doses of the xylanase enzymes according to SEQ ID NO: 1 and 2 in a water bath at a temperature of 84°C for 2 hours and with different doses of the xylanase enzyme according to SEQ ID NO: 3 in a water bath at a temperature of 40°C for 16 hours.

After the incubation, the whole stillage was directly filtrated with a Steriflip system that uses a 60pm nylon membrane. This filtration mimics the solid/liquid separation using a decanter centrifuge in an ethanol plant and allows to separate the wet cake that stays on top of the membrane, from the thin stillage that was further characterized. In this thin stillage, the moisture content was determined by weighing before and after drying in order to estimate the % dry solids content. The amount of nitrogen in the dried material was measured with a LECO analyzer and the total amount of proteins was calculated by multiplying the nitrogen content with the factor 6.25. The amount of solids and proteins obtained with xylanase treatment was compared to the negative control (no enzyme treatment) and increase in total solids and proteins in the thin stillage was calculated.

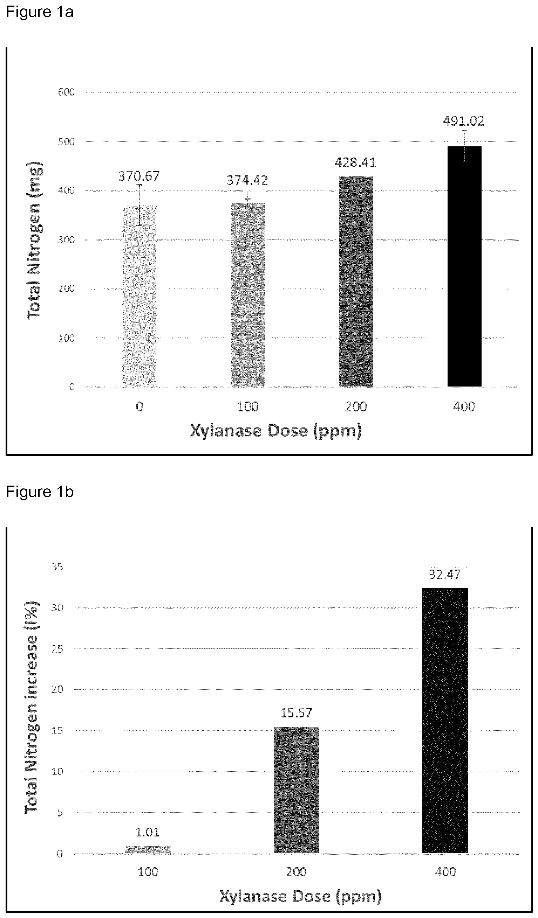

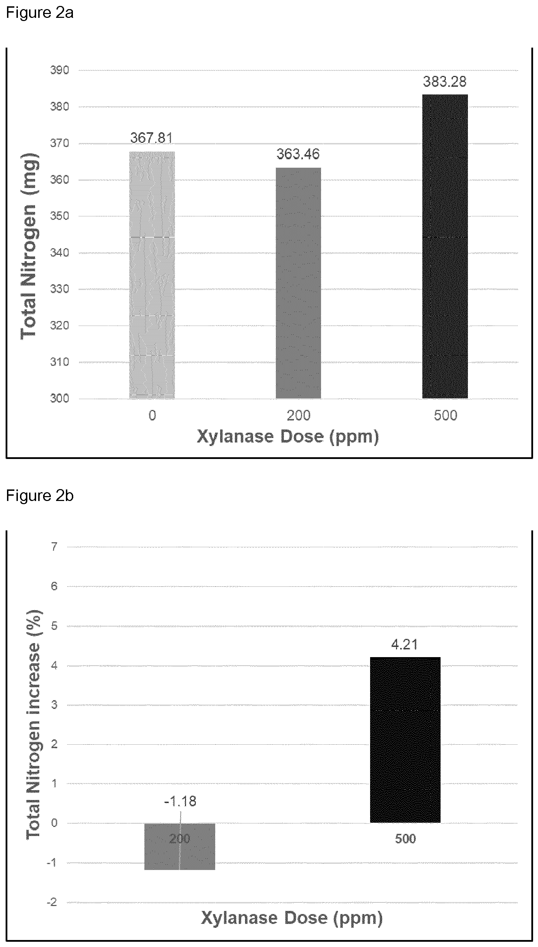

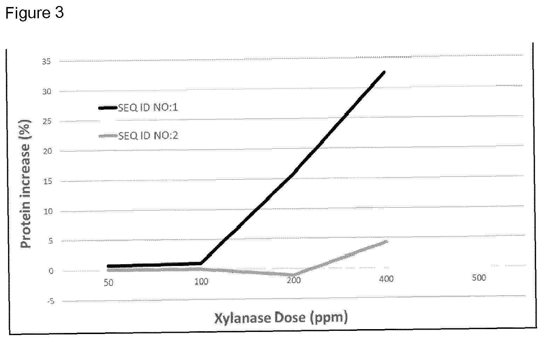

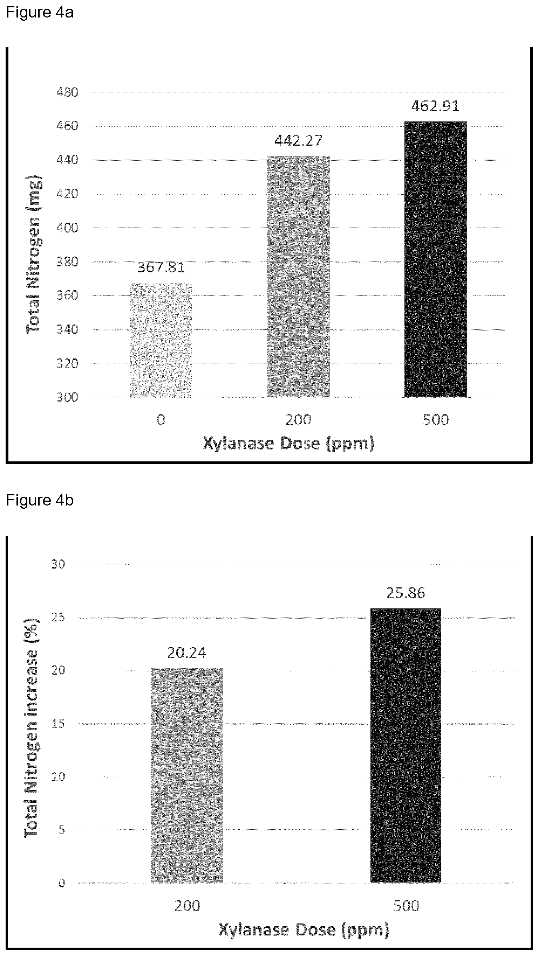

Figures 1 to 3 show that treatment of whole stillage with the GH10 xylanase according to SEQ ID NO: 1 increases the amount of nitrogen and consequently the protein content in thin stillage, whereas treatment of whole stillage with the same amounts of the GH11 xylanase according to SEQ ID NO: 2 does not significantly increase the amount of nitrogen and consequently the protein content in thin stillage. The treatment of thin stillage with the GH10 xylanase Rohalase also leads to an increase in the amount of nitrogen and consequently the protein content in thin stillage (see Figure 4).

## Claims

1. Method for increasing the solids content in thin stillage, comprising the steps of:
(a) adding a xylanase to whole stillage, thereby providing a mixture of whole stillage and xylanase; and
(b) incubating the mixture of whole stillage and xylanase; and
(c) separating the whole stillage into thin stillage and wet cake.

2. Method according to claim 1, wherein the solids comprise protein.

3. Method according to claim 1 or 2, wherein the mixture of whole stillage and xylanase is incubated at a temperature of at least 50°C.

4. Method according to any one of the preceding claims, wherein the mixture of whole stillage and xylanase is incubated for one to 24 hours.

5. Method according to any one of the preceding claims, wherein the xylanase is a thermostable xylanase.

6. Method according to any one of the preceding claims, wherein the xylanase is a GH10 xylanase.

7. Method according to any one of the preceding claims, wherein the xylanase has an amino acid sequence which is at least 80% to the amino acid sequence according to SEQ ID NO: 1.

8. Method according to any one of the preceding claims, wherein the xylanase is the only enzyme added to the whole stillage.

9. Method according to any one of the preceding claims, wherein the whole stillage is derived from a process of producing a fermentation product from a starch-containing material.

10. Method according to any one of the preceding claims, wherein the whole stillage is obtained by the steps of:
(a) liquefying a starch-containing material;
(b) fermenting the liquefied starch-containing material with a fermenting organism to produce a fermentation product, preferably in a simultaneous saccharification and fermentation process; and
(c) distilling the fermentation product to form the whole stillage.

11. Method according to claim 9 or 10, wherein the starch-containing material is a cereal, preferably is selected from the group consisting of corn, wheat, barley, cassava, sorghum, rye or potato.

12. Method according to any one of claims 9 to 11, wherein the fermentation product is an alcohol, preferably is ethanol.

13. Method according to any one of the preceding claims, wherein the whole stillage is separated into thin stillage and wet cake by use of paddle screen.

14. Method according to any one of the preceding claims, further comprising a step (d) of isolating protein from thin stillage.

15. Use of a xylanase for increasing the solids content, preferably the protein content, in thin stillage.
